(19) Europäisches Patentamt
European Patent Office
Office européen des brevets

(11) Numéro de publication : **0 080 944**
**B1**

(12) # FASCICULE DE BREVET EUROPÉEN

(45) Date de publication du fascicule du brevet : **28.08.85**

(51) Int. Cl.⁴ : **C 07 D 501/36**, A 61 K 31/545

(21) Numéro de dépôt : **82402151.3**

(22) Date de dépôt : **26.11.82**

(54) **Nouveaux dérivés de céphalosporines substitués en position 3 par un groupe hétérocycle thiométhyl, procédé de préparation desdits composés et compositions pharmaceutiques en contenant.**

(30) Priorité : **01.12.81 FR 8122506**

(43) Date de publication de la demande : **08.06.83 Bulletin 83/23**

(45) Mention de la délivrance du brevet : **28.08.85 Bulletin 85/35**

(84) Etats contractants désignés : **AT BE CH DE FR GB IT LI LU NL SE**

(56) Documents cités : **FR-A- 2 387 234**

(73) Titulaire : **SANOFI, société anonyme**
**40, Avenue George V**
**F-75008 Paris (FR)**

(72) Inventeur : **Labeeuw, Bernard**
**49, Lotissement des Genêts Avenue des Moulins**
**F-34000 Montpellier (FR)**
Inventeur : **Salhi, Ali**
**639 rue de Valène**
**F-34980 Saint-Gely du Fesc (FR)**

(74) Mandataire : **Gillard, Marie-Louise**
**Cabinet Beau de Loménie 55, Rue d'Amsterdam**
**F-75008 Paris (FR)**

**EP 0 080 944 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne de nouveaux dérivés de céphalosporines, un procédé pour leur préparation et des compositions pharmaceutiques renfermant lesdits dérivés de céphalosporines en tant qu'ingrédients actifs.

Plus particulièrement l'invention se réfère à de nouvelles céphalosporines substituées en position 3 par un groupe Het-S-$CH_2$ dans lequel Het désigne un hétérocycle à 5 chaînons comportant au moins 2 atomes d'azote et éventuellement un atome de soufre ou un hétérocycle à 6 chaînons comportant 1 atome d'azote.

Le brevet belge 866 038 cite ou décrit entre autres une série de sulfoxydes de céphalosporines répondant à la formule générale

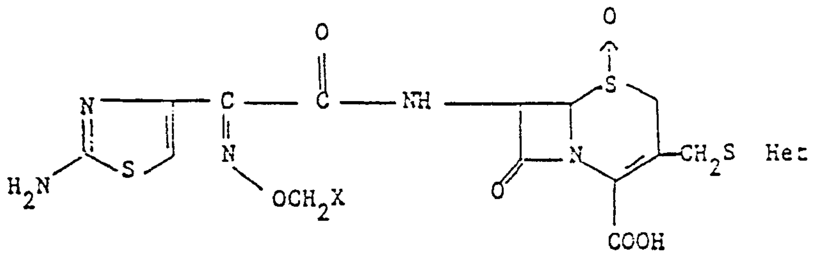

(I)

X = H, COOH

dans laquelle Het représente un hétérocycle à 5 ou 6 chaînons et notamment un triazole-1,2,3, un triazole-1,3,4, un thiadiazole-1,3,4, un tétrazole ou un groupe pyridyl-2 éventuellement substitués.

Les céphalosporines de formule (I) sont censées posséder de façon générale une très forte activité bactérienne contre les bactéries Gram positives et Gram négatives et être efficaces contre les staphylocoques producteurs de pénicillinase.

La présente invention a pour objet de nouvelles céphalosporines qui possèdent un profil bactérien très différent de celui des composés du brevet cité précédemment. En effet les composés de l'invention possèdent une activité remarquable sur les entérobactéries y compris celles productrices de β-lactamases alors qu'ils sont très faiblement actifs sur les staphylocoques.

Ces nouvelles céphalosporines répondent à la formule générale

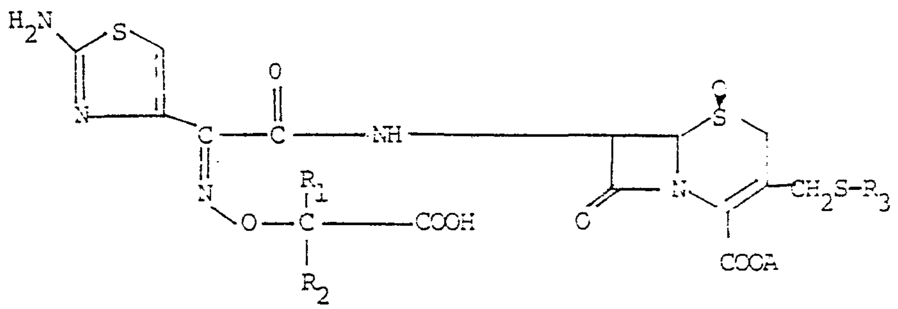

(II)

dans laquelle

$R_1$ et $R_2$ considérés isolément représentent chacun un groupe méthyle ou encore

$R_1$ et $R_2$ considérés ensemble représentent un groupe 1,3 propylène ;

$R_3$ représente un hétérocycle de formule

a) 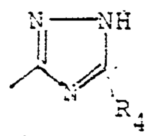 avec $R_4$ = H, $NH_2$

b) 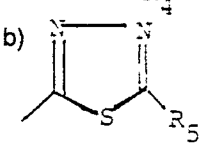 avec $R_5$ = $NH_2$, SH

c) 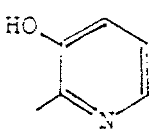

A représente l'hydrogène, un cation ou un ester ou hémiacétal facilement hydrolysable ou métaboliquement labile et pharmaceutiquement acceptables.

Dans la présente demande :

— le terme « cation » désigne un ion alcalin ou alcalino-terreux, de préférence les ions sodium, potassium ou calcium, ou bien le dérivé « ammonium » résultant par protonation d'une amine organique pharmaceutiquement acceptable telle que l'éthylènediamine, l'éthanolamine, la trométhamine et similaires, ou d'un aminoacide tel que la lysine, l'arginine ou l'acétylcystéine, pour former des sels d'addition ;

— le terme ester ou hémiacétal facilement hydrolysable ou métaboliquement labile et pharmaceutiquement acceptable désigne des radicaux tels que phtalidyle, pivaloyloxyméthyle, acétoxyméthyle, éthoxycarbonyloxyméthyle, 1-(éthoxycarbonyloxy)-éthyle, acétonyle, α-méthoxy, α-carbométhoxyméthyle, carbométhoxyméthyle, carbétoxyméthyle et similaires.

L'invention concerne également un procédé de préparation des composés de formule (II)

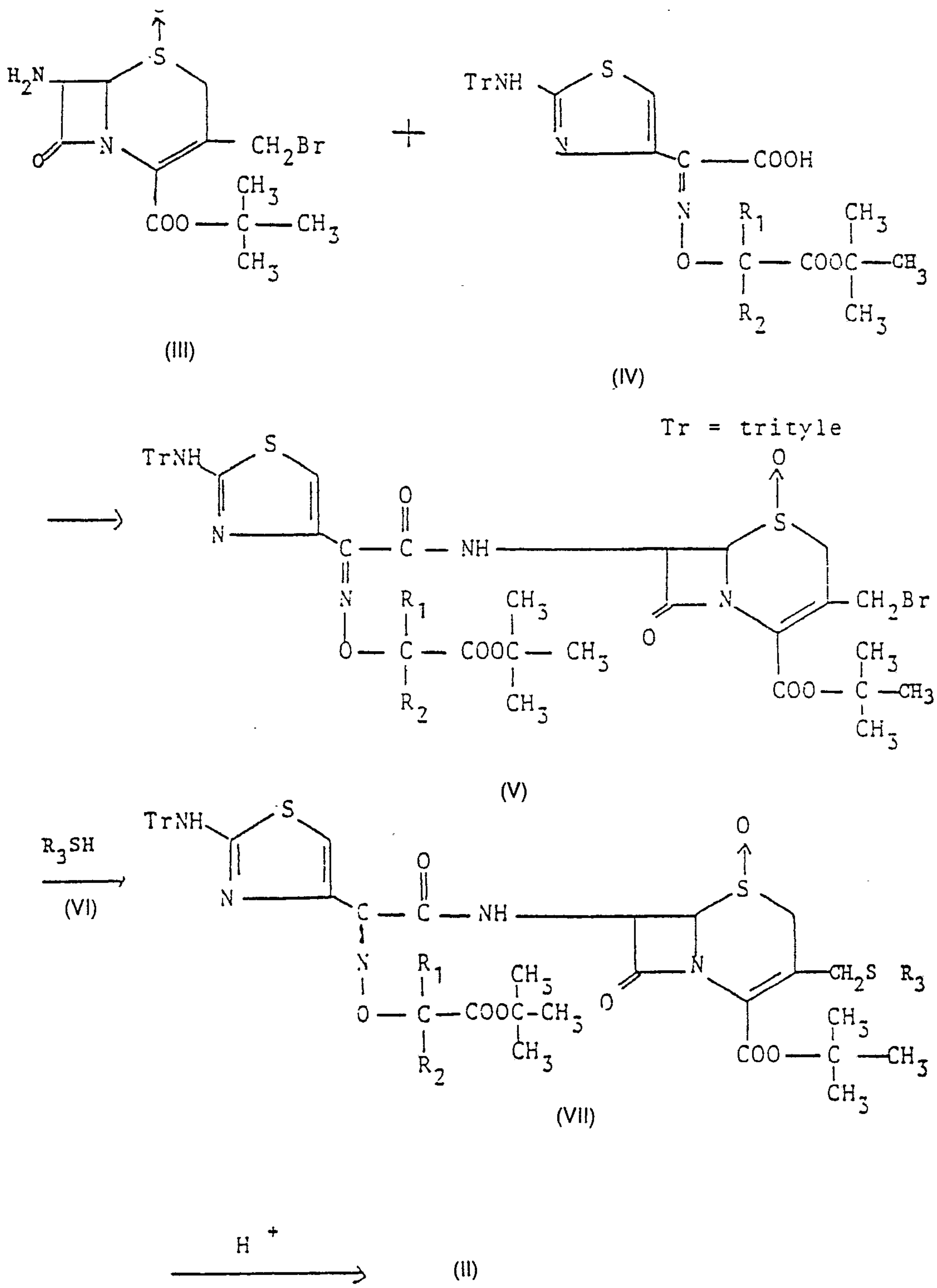


(III) (IV) (V) (VII) (II)

La première étape consiste à acyler l'amino-7 bromométhyl-3 céphème-3 carboxylate de tertiobutyle S-oxyde-1 (III) par l'acide (IV). Avant d'effectuer la réaction d'acylation, il est souhaitable de substituer le groupe amino de l'acide (IV) par un groupe protecteur facile à éliminer ultérieurement. On peut utiliser les groupes habituellement utilisés en synthèse organique pour la protection des amines et en particulier le groupe trityle.

Pour effectuer la réaction d'acylation, il est nécessaire de procéder à l'activation du groupe carboxyle du composé (IV) de préférence par transformation de l'acide en anhydride par action d'un carbodiimide tel que le dicyclohexylcarbodiimide.

La réaction d'activation est effectuée au sein d'un solvant organique convenable tel que le tétrahydrofuranne à une température comprise entre 0 et 50 °C et, de préférence, à température ambiante. La réaction d'activation est éventuellement facilitée par addition d'un dérivé hydroxylé tel que l'hydroxy-1 benzotriazole.

La solution du réactif d'acylation ainsi obtenu, débarrassée par filtration de la dicyclohexylurée formée, est ajoutée à une solution du composé (III) dans un solvant tel que le diméthylformamide. L'addition des deux réactifs peut aussi s'effectuer dans l'ordre inverse.

Par action sur le composé (V) ainsi obtenu d'un thiol $R_3SH$ (VI) éventuellement substitué sur l'hétérocycle, on obtient le composé (VII). La réaction est effectuée par contact des deux réactifs au sein d'un solvant convenable tel que le diméthylformamide ou le N,N-diméthylacétamide à une température comprise entre 0 et 50 °C et de préférence à température ambiante. On opère en présence d'un agent alcalin tel que le triéthylamine ou le bicarbonate de potassium.

Le produit (VII) est isolé par dilution avec de l'eau puis purifié par les méthodes usuelles et en particulier par chromatographie sur gel de silice.

On peut également remplacer le thiol par son sel de sodium. On utilise les mêmes solvants de réaction et on opère alors sans agent alcalin.

Enfin pour aboutir aux composés (II) les groupes protecteurs sur l'amine et les fonctions carboxyle sont éliminés simultanément par un procédé connu, en particulier par hydrolyse en milieu acide en utilisant un acide minéral ou organique tel que l'acide chlorhydrique dans l'acide formique ou l'acide trifluoracétique.

En ce qui concerne les matières premières utilisées dans ce procédé les composés (III) et les composés (IV) ainsi que leurs dérivés dans lesquels le groupe aminé est bloqué par un groupe protecteur sont connus.

Les composés (II) de l'invention dans lesquels A est autre que H s'obtiennent à partir des composés (II) dans lesquels A est H par des réactions connues en elles-mêmes. Ainsi, les sels minéraux sont obtenus par action sur les composés (II) dans lesquels A = H d'une base minérale telle que la soude ou la potasse ou le bicarbonate de sodium en quantité équimoléculaire. La réaction est effectuée dans un solvant tel que l'eau ou l'éthanol et le sel obtenu est isolé par évaporation de la solution.

Les sels de bases organiques ou d'aminoacides sont obtenus par aciton, sur une solution de l'acide (II ; A = H) dans un solvant ou un mélange de solvants convenables, d'une quantité équimoléculaire de la base organique. Le sel est isolé par précipitation avec l'éther.

Les esters sont obtenus par les procédés connus d'estérification, par exemple on utilisera avantageusement l'action d'un dérivé halogéné sur un sel tel que le sel de sodium de l'acide. On réalisera de préférence la réaction dans un solvant capable de dissoudre le dérivé acide de départ par exemple dans le diméthylformamide.

Les isomères de forme syn et anti s'obtiennent par un choix convenable des réactifs.

Les exemples suivants permettent de mieux comprendre la portée de l'invention.

Ainsi qu'il est habituel dans cette famille de composés, les produits suivant l'invention ne présentent pas de point de fusion net mais seulement des points de décomposition ne permettant pas de les caractériser.

Les produits seront donc caractérisés par leur spectre de résonance magnétique nucléaire enregistré à 60 MHz, l'étalon interne étant l'hexaméthyldisiloxanne.

Les abréviations suivantes seront utilisées :

— S : singulet
— D : doublet
— T : triplet
— Q : quadruplet
— D de D : doublet de doublet
— S. e. : singulet élargi
— M : multiplet
— AB : système AB
— J : représente la constante de couplage.

De plus les microanalyses élémentaires ont été effectuées dans chaque cas et sont en accord avec les formules indiquées.

Exemple 1

Acide [(amino-2 thiazolyl-4)-2 (carboxy-2 propyl-2 oxyimino)-2 acétamido]-7 (1H triazole-1,2,4 yl-3 thiométhyl)-3 céphème-3 carboxylique-3 S-oxyde-1 Isomère syn (CM 40 765)

$R_1 = R_2 = CH_3$ ; A = H ; $R_3$ = 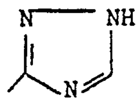 (II)

a) [(tritylamino-2 thiazolyl-4)-2 (t-butoxycarbonyl-2 propyl-2 oxyimino)-2 acétamido]-7 bromométhyl-3 céphème-3 carboxylate de tertiobutyle-4 S-oxyde-1 isomère syn.

$R_1 = R_2 = CH_3$ (V)

A une solution de 5 g de chlorhydrate d'amino-7 bromométhyl-3 céphème-3 carboxylate de tertiobutyle-4 S-oxyde-1 dans 90 ml de chlorure de méthylène, on ajoute 1,72 ml de triéthylamine, 7,57 g d'acide (tritylamino-2 thiazolyl-4)-2 (t-butoxycarbonyl-2 propyl-2 oxyimino)-2 acétique, 2,84 g de dicyclohexylcarbodiimide et 0,1 g d'hydroxybenzotriazole. On agite le mélange pendant 15 heures à température ambiante puis on filtre la dicyclohexylurée formée.

Après évaporation du solvant, on chromatographie le résidu sur une colonne de gel de silice (250 g). On éluant avec un mélange hexane-acétate d'éthyle 50-50 (vol/vol), on obtient 4,3 g du produit attendu.

Spectre de RMN (en solution dans le diméthylsulfoxyde deutérié).

1 H à 8,70 ppm (NH-Trit, S) — 1 H à 8,07 ppm (NH-CO, D, J = 9 Hz) — 15 H à 7,25 ppm (H trit, S) — 1 H à 6,72 ppm (H thiazole, S) — 1 H à 5,88 ppm ($H_7$, D de D, $J_1$ = 9 Hz, $J_2$ = 4 Hz) — 1 H à 4,96 ppm ($H_6$, D, J = 4 Hz) — 2 H à 4,50 ppm ($CH_2Br$, AB, $J_{AB}$ = 12 Hz) — 2 H à 3,77 ppm ($CH_2$ en 2, S. e.) — 9 H à 1,45 ppm

($CH_2$ en 2, S. e.) - 9 H à 1,45 ppm 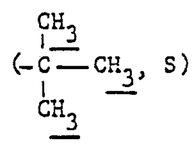 — 6 H à 1,37 ppm

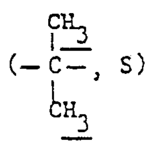 — 9 H à 1,27 ppm 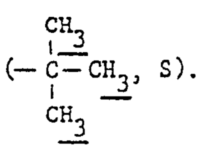.

b) [(tritylamino-2 thiazolyl-4)-2 (t-butoxycarbonyl-2 propyl-2 oxyimino)-2 acétamido]-7 (1H triazole-1,2,4 yl-3 thiométhyl)-3 céphème-3 carboxylate de t-butyle-4 S-oxyde-1 isomère syn.

$R_1 = R_2 = CH_3$ ; $R_3$ = 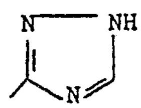 (VII)

A une solution de 2,8 g du dérivé bromé obtenu ci-dessus dans 20 ml de N,N-diméthylacétamide, on ajoute 0,308 g de mercapto-3 1H triazole-1,2,4 puis 0,4 ml de triéthylamine. Après 3 heures d'agitation à température ambiante, on évapore le solvant sous vide et dissout le résidu dans 80 ml de chlorure de méthylène. On chromatographie sur une colonne de 120 g de gel de silice. En éluant avec de l'acétate d'éthyle, on obtient 2,3 g du produit attendu.

c) CM 40 765.

On agite pendant 30 minutes à température ambiante 1,06 g du produit précédent dans 10 ml d'acide trifluoracétique. On évapore sous vide jusqu'à 5-6 ml puis précipite par addition de 200 ml d'éther anhydre. On essore le précipité, lave avec de l'éther anhydride et sèche. On répète une seconde fois l'opération précédente et obtient de la même façon 0,6 g du produit attendu.

Spectre de RMN

5 H entre 9 et 10,5 ppm (NH triazole, $NH_2$, 2 COOH, S. e.) — 2 H à 8,40 ppm (NHCO, H triazole, M) — 1 H à 6,87 ppm (H thiazole, S) — 1 H à 5,97 ppm ($H_7$, M) — 1 H à 4,92 ppm ($H_6$, D, J = 4 Hz) — 1 H à 4,5 ppm ($CH_2S$—, A de AB, $J_{AB}$ = 13 Hz) — 1 H à 4,30 ppm ($CH_2S$, B de AB, $J_{AB}$ = 13 Hz) — 2 H à 3,80 ppm ($CH_2S \rightarrow$ 0, S. e.) — 6 H à 1,45 ppm

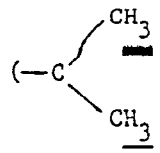 , S).

Exemple 2

Acide [(amino-2 thiazolyl-4)-2 (carboxy-2 propyl-2 oxyimino)-2 acétamido]-7 (amino-2 thiadiazol-1,3,4 yl-5 thiométhyl)-3 céphème-3 carboxylique-4 S-oxyde-1 isomère syn (CM 40 803).

$R_1 = R_2 = CH_3$ ; A = H ; $R_3$ = 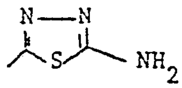 (II)

a) [(tritylamino-2 thiazolyl-4)-2 (t-butoxycarbonyl-2 oxyimino)-acétamido]-7 (amino-2 thiadiazol-1,3,4 yl-5 thiométhyl)-3 céphème-3 carboxylate de t-butyle-4 S-oxyde-1 isomère syn.

$R_1 = R_2 = CH_3$ ; $R_3$ = 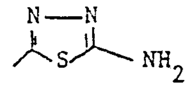 (VII)

On agite pendant 16 heures à température ambiante le mélange de 1 g du dérivé bromé de l'exemple 1a), 0, 18 g d'amino-2 mercapto-5 thiadiazole-1,3,4 et 0,12 g de bicarbonate de potassium dans 10 ml de diméthylformamide. On évapore le solvant sous vide et dissout le résidu dans du chlorure de méthylène. On lave la solution avec de l'eau puis avec une solution aqueuse saturée de chlorure de sodium. On sèche sur sulfate de magnésium puis concentre la solution à 5 ml. On chromatographie sur une colonne de 25 g de gel de silice. En éluant avec un mélange acétate d'éthylehexane 90-10 (vol/vol) on obtient 1 g du produit attendu.

b) CM 40 803.

On agite pendant 45 minutes à 20 °C, le mélange de 0,55 g du produit protégé obtenu ci-dessus et 6 ml d'acide trifluoracétique. On concentre sous vide à 3 ml environ puis précipite par addition d'éther. On essore le solide et sèche sur anhydride phosphorique. On obtient 0,39 g du produit attendu.

Spectre de RMN

1 H à 8,5 ppm (NHCO, D, J = 9 Hz) — 6 H entre 6,5 et 8,5 ppm (2 $NH_2$, 2 COOH, M) — 1 H à 6,90 ppm (H thiazole, S) — 1 H à 5,97 ppm ($H_7$, M) — 1 H à 4,96 ppm ($H_6$, D, J = 4 Hz) — 1 H à 4,45 ppm ($CH_2S$, A de AB, $J_{AB}$ = 13 Hz) — 1 H à 3,90 ppm ($CH_2S$, B de AB, $J_{AB}$ = 13 Hz) — 2 H à 3,85 ppm ($CH_2S \rightarrow O$, S. e.) — 6 H à 1,45 ppm

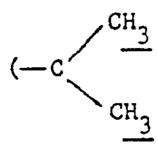 , S).

Exemples 3 à 5

On opère comme dans l'exemple 2a) à partir du dérivé bromé de l'exemple 1a) en faisant varier la nature du thiol utilisé.

En effectuant ensuite la déprotection des produits obtenus ainsi qu'indiqué dans l'exemple 2b), on obtient les différents composés (II) réunis dans le tableau 1.

Tableau 1

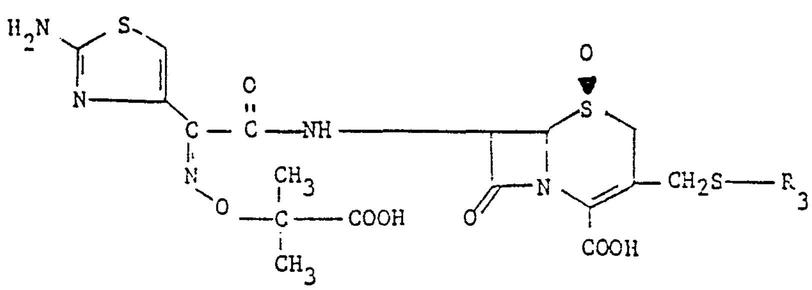

Tableau 1 (Suite)

| n° exemple | n° de code du produit | $R_3$ | Spectre de RMN |
|---|---|---|---|
| 3 | 40.804 | 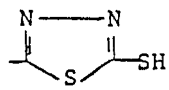 | 1 H à 8,40 ppm (NHCO, D, J=9 Hz - 4 H entre 8,5 et 10 ppm ($NH_2$, 2 COOH) - 1 H à 6,83 ppm (H thiazole, S) - 1 H à 5,96 ppm ($H_7$, D de D, $J_1$=9 Hz, $J_2$= 4Hz) - 1 H à 4,93 ppm ($H_6$, D, J=4 Hz) - 1H à 4,60 ppm ($CH_2S$, A de AB, $J_{AB}$=13 Hz) - 1 H à 4,05 ppm ($CH_2S$, B de AB, $J_{AB}$= 13 Hz) - 2H à 3,81 ppm ($CH_2S{\rightarrow}O$, S. e.)- 6 H à 1,42 ppm (- 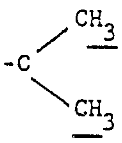 , S). |
| 4 | 40 805 | 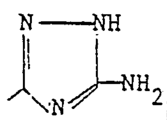 | 7 H entre 8 et 10 ppm (NH triazole, 2 $NH_2$, 2 COOH, S.e.) - 1 H à 8,45 ppm (NHCO, D, J=9 Hz) - 1H à 6,85 ppm (H thiazole, S) - 1 H à 5,97 ppm ($H_7$, D de D, $J_1$=9 Hz, $J_2$=4 Hz) - 1 H à 4,95 ppm ($H_6$, D, J=4Hz) - 1 H à 4,30 ppm ($CH_2S$, A de AB, $J_{AB}$=13 Hz) - 1 H à 3,90 ppm ($CH_2S$, B de AB, $J_{AB}$= 13 Hz) - 2 H à 3,85 ppm ($CH_2S{\rightarrow}O$, S.e.)- 6 H à 1,45 ppm ( 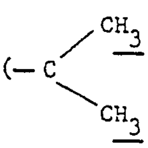 , S). |
| 5 | 40.953 | 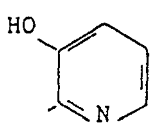 | 5 H à 9,0 ppm (2 COOH, $NH_2$, OH M) - 1H à 8,43 ppm (NHCO, D, J=9Hz) - 1 H à 7,87 ppm ($H_6$ pyridine, M) - 3 H à 6,95 ppm ($H_4$ et $H_5$ pyridine, H thiazole, M) - 1 H à 5,95 ppm ($H_7$, D de D, $J_1$=9 Hz, $J_2$=5 Hz) - 1 H à 4,95 ppm ($H_6$, D, J=5 Hz) - 1 H à 4,60 ppm ($CH_2S$, A de AB, $J_{AB}$=13 Hz) - 3 H à 3,80 ppm ($CH_2S{\rightarrow}O$ et $CH_2S$, B de AB, M) - 6 H à 1,45 ppm (- 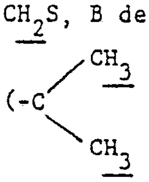 , S). |

Exemple 6

Acide [(amino-2 thiazolyl-4)-2 (carboxy-1 cyclobutyl oxyimino)-2 acétamido]-7 (hydroxy-3 pyridinyl-2 thiométhyl)-3 céphème-3 carboxylique-4 S-oxyde-1 isomère syn (CM 41 646).

$R_1 + R_2 = (CH_2)_3$ ; $R_3$ = 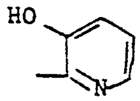 ; A = H

a) [(tritylamino-2 thiazolyl-4)-2 (t-butyoxycarbonyl-1 cyclobutyl-1 oxyimido)-2 acétamido]-7 bromométhyl-3 céphème-3 carboxylate de t-butyle-4 S-oxyde-1 isomère syn.

$$R_1 + R_2 = (CH_2)_3 \qquad (V)$$

A une solution de 4,4 g de chlorhydrate d'amino-7 bromométhyl-3 céphème-3 carboxylate de t-butyle S-oxyde-1 dans 70 ml de chlorure de méthylène anhydre, on ajoute sous atmosphère d'azote 1,5 ml de triéthylamine, 5,1 g d'acide (tritylamino-2 thiazolyl-4)-2 (t-butoxycarbonyl-1 cyclobutyl-1 oxyimino)-2 acétique isomère syn, 2,4 g de dicyclohexylcarbodiimide et 0,1 g d'hydroxy-1 benzotriazole. On agite durant 1 h à température ambiante, puis on filtre la dicyclohexylurée formée et concentre la solution à 20 ml sous vide.

On chromatographie sur une colonne de gel de silice (150 g). Par élution avec le mélange hexane-acétate d'éthyle 40-60 (vol/vol), on obtient, après évaporation du solvant, 4,8 g du produit attendu.

Spectre de RMN

1 H à 7,90 ppm (NHCO, D, J = 9 Hz) — 15 H à 7,26 ppm (H aromatiques, S) — 1 H à 6,97 ppm (NH-trityle, S. e.) — 1 H à 6,65 ppm (H thiazole, S) — 1 H à 6,18 ppm ($H_7$, D de D, $J_1$ = 9 Hz, $J_2$ = 4,5 Hz) — 2 h à 3,4 ppm ($CH_2S \rightarrow O$, S. e.) — 6 H entre 1,5 et 2,6 ppm (cyclobutyle, M) — 9 H à 1,46 ppm

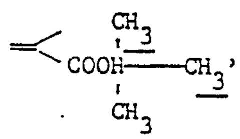 S) — 9 H à 1,36 ppm

( 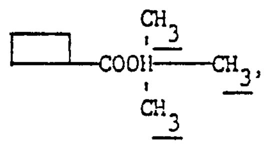 , S).

b) [Tritylamino-2 thiazolyl-4)-2 t-butoxycarbonyl-1 cyclobutyl-1 oxyimino)-2 acétamido]-7 (hydroxy-3 pyridinyl-2 thiométhyl)-3 céphème-3 carboxylate de t-butyle-4 S-oxyde-1 isomère syn.

A une solution de 0,164 g d'hydroxy-3 mercapto-2 pyridine dans 6 ml de diméthylformamide, on ajoute 0, 128 g de bicarbonate de potassium, puis 1 g du dérivé bromé obtenu ci-dessus. On agite pendant 16 h à température ambiante, puis verse dans 50 ml d'eau glacée. On essore le précicpité et lave avec de l'eau. On redissout le solide dans le chlorure de méthylène, sèche la solution sur sulfate de magnésium et concentre à 5 ml. On chromatographie sur une colonne de 20 g de gel de silice. En éluant avec le mélange hexane-acétate d'éthyle 40-60 (vol/vol), on obtient 0,85 g du produit attendu.

c) CM 41 646.

On laisse à température ambiante pendant 45 min la solution de 0,8 g du produit obtenu en b) dans 8 ml d'acide trifluoracétique. On évapore à siccité sous vide et triture le résidu avec de l'éther. On essore le solide et sèche sous vide en présence d'anhydride phosphorique pour obtenir 0,5 g du produit attendu.

Spectre de RMN

1 H à 10,40 ppm (OH, S. e.) — 1 H à 8,70 ppm (NHCO, D, J = 8 Hz), 1 H à 7,80 ppm ($H_6'$ pyridine, D, J = 5 Hz) — 2 H à 7,10 ppm ($NH_2$, S. e.) — 2 H à 6,95 ppm ($H_4'$ et $H_5'$ pyridine, M) — 1 H à 6,80 ppm (H thiazole, S) — 1 H à 5,90 ppm ($H_7$, D de D, $J_1$ = 8 Hz, $J_2$ = 5 Hz) — 1 H à 4,95 ppm ($H_6$, D, J = 5 Hz) — 1 H à 4,65 ppm ($CH_2S$, D, J = 14 Hz) — 3 H à 3,80 ppm ($CH_2S \rightarrow O$ et $CH_2S$, M) — 4 H à 2,40 ppm

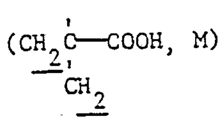 — 2 H à 1,90 ppm ( 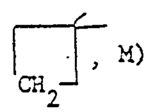 , M).

Les produits (II) ont été étudiés en ce qui concerne leurs propriétés pharmacologiques et plus spécialement leur action bactériostatique.

L'action bactériostatique in vitro a été déterminée en milieu solide par la méthode des dilutions. Les

résultats obtenus sont exprimés en concentrations minimales inhibitrices (CMI-µg/ml) et concernent différentes souches d'entérobactéries et de Pseudomonas.

A titre de comparaison, on a ajouté les résultats obtenus avec deux produits voisins décrits dans l'art antérieur (brevet belge n° 866 038), à savoir

— l'acide [(amino-2 thiazolyl-4)-2 carboxyméthoxyimino-2 acétamido]-7 (pyridyl-2 thiométhyl)-3 céphème-3 carboxylique-4 S-oxyde-1 isomère syn :

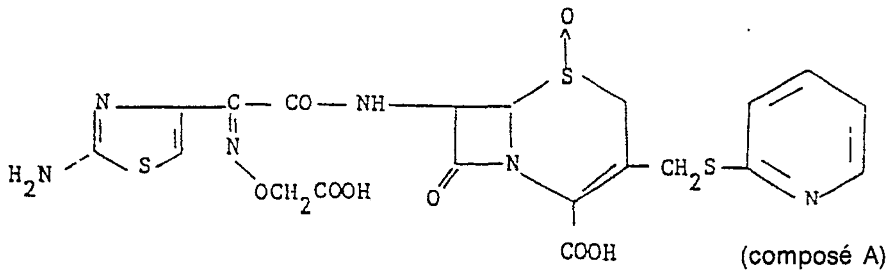

(composé A)

— l'acide [(amino-2 thiazolyl-4)-2 méthoxyimino-2 acétamido]-7 (triazol-1,2,4 yl-3 thiométhyl)-3 céphème-3 carboxylique-4 S-oxyde-1 isomère syn.

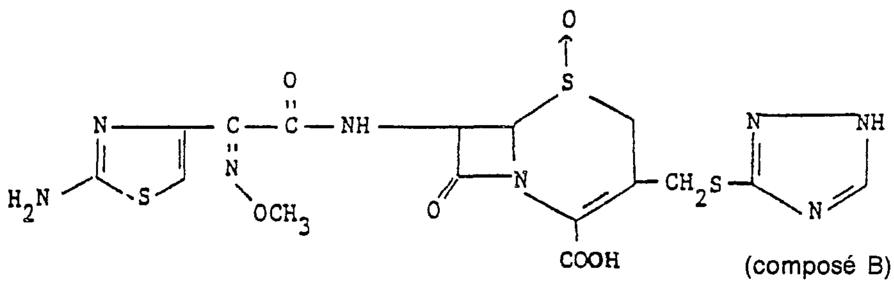

(composé B)

Les résultats obtenus sont rassemblés dans le tableau 2. Ces résultats montrent une activité intéressante des produits selon l'invention sur des souches habituellement peu sensibles aux antibiotiques de la famille des céphalosporines, à savoir les Entérobactéries et les Pseudomonas.

Vis-à-vis des produits de référence A et B, les produits (II) montrent une activité surprenante sur les souches de Pseudomonas, une bonne activité sur Enterobacter tout en présentant une activité au moins égale à celle des produits de référence vis-à-vis des Proteus, Serratia et Escherichia coli.

Par ailleurs, les essais effectués sur les animaux n'ont mis en évidence aucune toxicité des produits selon l'invention.

Les produits de l'invention peuvent donc être utilisés comme antibiotiques en médecine humaine ou vétérinaire. Ils peuvent être utilisés dans toutes les infections bactériennes à germes sensibles.

Les compositions pharmaceutiques sont réalisées à partir des composés (II) sous leur forme acide ou, lorsque leur solubilité est insuffisante, sous forme d'un sel.

Les compositions pharmaceutiques peuvent être solides ou liquides et se présenter, par exemple, sous forme de comprimés, gélules, granulés, pommades, crèmes, gels ou préparations injectables.

La posologie peut varier dans de larges proportions, en particulier suivant le type et la gravité de l'infection à traiter et suivant le mode d'administration. Le plus souvent, chez l'adulte par voie injectable, elle est comprise entre 0,250 g et 4 g par jour.

A titre d'exemple de composition pharmaceutique contenant un des produits de l'invention, on peut préparer des ampoules injectables contenant :

| | |
|---|---|
| CM 40 953 | 1 g |
| Eau pour préparation injectable | 5 ml |
| Carbonate de sodium qs pH = 6,5 | |

(voir tableau 2 p. 10)

Tableau 2

| Souche \ Produit | 40765 | 40303 | 40804 | 40805 | 40953 | 41646 | A | B |
|---|---|---|---|---|---|---|---|---|
| Pseudomonas RL 112 | 4 | 4 | 8 | 4 | 8 | 16 | 256 | 512 |
| Pseudomonas 8203 | 8 | 4 | 8 | 4 | 16 | 16 | 256 | 256 |
| Pseudomonas 526 | 8 | 4 | 8 | 4 | 16 | 16 | >256 | 512 |
| Pseudomonas A 22 IP | 8 | 8 | 8 | 8 | 16 | 32 | >256 | 512 |
| Pseudomonas 103 IFE | 8 | 4 | 8 | 8 | 16 | 16 | >256 | 256 |
| Proteus 1510 | 0,125 | ≤0,125 | ≤0,125 | ≤0,125 | ≤0,125 | - | 0,25 | 0,25 |
| Serratia RL 72 | 4 | 1 | 4 | 4 | 8 | 8 | 32 | 8 |
| Enterobacter P 99 | 8 | 2 | 4 | 2 | 8 | - | 16 | 32 |

**Revendications** (pour les Etats contractants : BE, CH, DE, FR, GB, IT, LI, LU, NL, SE)

1. Nouvelles céphalosporines caractérisées en ce qu'elles répondent à la formule

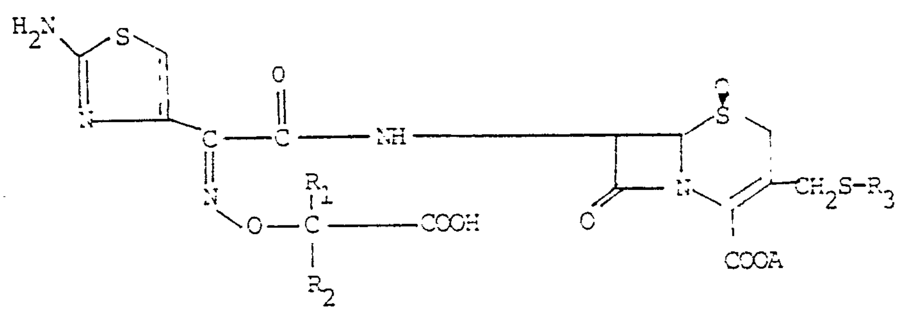

(II)

dans laquelle

$R_1$ et $R_2$ considérés isolément représentent chacun un groupe méthyle ou encore $R_1$ et $R_2$ considérés ensemble représentent un groupe 1,3 propylène ;

$R_3$ représente un hétérocycle de formule

a) 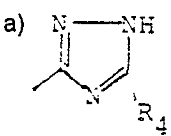 avec $R_4$ = H, $NH_2$

b) 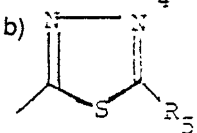 avec $R_5$ = $NH_2$, SH

c) 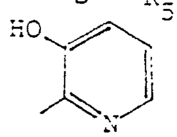

et A représente l'hydrogène, un cation ou un ester ou hémiacétal facilement hydrolysable ou métaboliquement labile et pharmaceutiquement acceptables.

2. Nouvelles céphalosporines selon la revendication 1, caractérisées en ce qu'elles sont sous la forme d'isomère syn ou d'isomère anti ou sous forme d'un mélange de ces isomères.

3. Médicaments à action antibactérienne, caractérisés en ce qu'ils contiennent au moins une nouvelle céphalosporine selon l'une des revendications 1 et 2.

4. Médicaments selon la revendication 3, caractérisés en ce qu'ils contiennent un produit de formule II dans laquelle $R_1$ et $R_2$ sont $CH_3$, A est H et $R_3$ est choisi parmi les groupes suivants

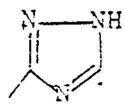 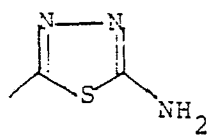 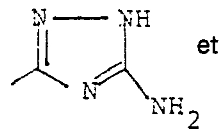 et 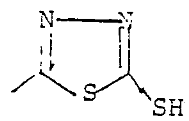

5. Médicaments selon la revendication 3, caractérisés en ce qu'ils contiennent un produit de formule II dans laquelle $R_1$ et $R_2$ sont $CH_3$, A est H et $R_3$ est OH

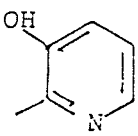

6. Procédé de préparation des nouvelles céphalosporines selon l'une des revendications 1 et 2, caractérisé en ce que l'on effectue les réactions successives suivantes

— on acyle l'amino-7 bromo-méthyl-3 céphème-3 carboxylate de tertiobutyl S-oxyde-1 sur un acide de formule

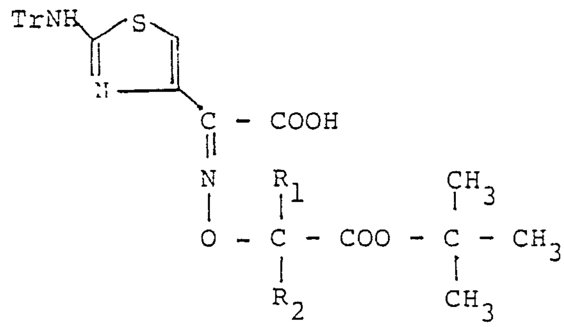

dans laquelle $R_1$ et $R_2$ ont la signification donnée dans la revendication 1 et Tr est un groupe protecteur, par exemple, trityle, ledit acide ayant été activé par transformation, par exemple en anhydride, ladite réaction d'acylation étant réalisée en solution par exemple dans le diméthylformamide,

— puis ont fait réagir le produit obtenu sur un thiol de formule $R_3SH$, la réaction étant effectuée dans un solvant convenable, tel que le diméthylformamide, à une température comprise entre 0 et 50 °C et de préférence en présence d'un agent alcalin,

— puis on enlève, par des méthodes connues comme par exemple l'hydrolyse, les groupes protecteurs situés sur les fonctions amine et acide du produit obtenu,

— puis éventuellement on transforme la fonction acide en sel ou ester en employant des procédés connus.

**Revendications** (pour l'Etat contractant AT)

1. Procédé pour l'obtention de céphalosporines répondant à la formule

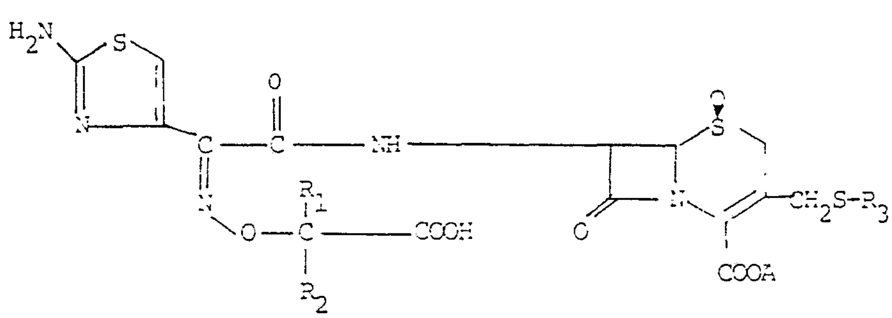

(II)

dans laquelle

$R_1$ et $R_2$ considérés isolément représentent chacun un groupe méthyle ou encore

$R_1$ et $R_2$ considérés ensemble représentent un groupe 1,3 propylène ;

$R_3$ représente un hétérocycle de formule

a) 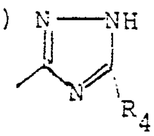 avec $R_4$ = H, $NH_2$

b) 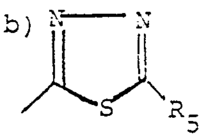 avec $R_5$ = $NH_2$, SH

c) 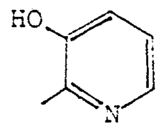

et A représente l'hydrogène, un cation ou un ester ou hémiacétal facilement hydrolysable ou métaboliquement labile et pharmaceutiquement acceptables, caractérisé en ce qu'il consiste à

1) acyler l'amino-7 bromo-méthyl-3 céphème-3 carboxylate de tertiobutyl S-oxyde-1 sur un acide de formule

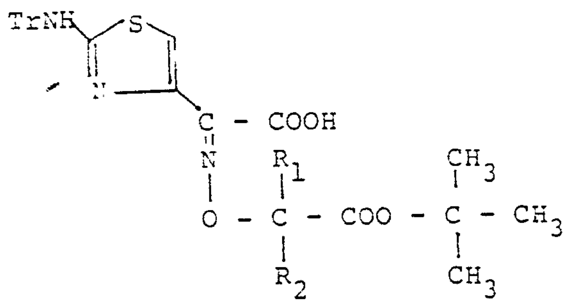 (IV)

dans laquelle $R_1$ et $R_2$ ont la signification donnée ci-dessus et Tr est un groupe protecteur, par exemple, trityle, ledit acide ayant été activé par transformation, par exemple en anhydride, ladite réaction d'acylation étant réalisée en solution par exemple dans le diméthylformamide,

2) à faire réagir le produit obtenu sur un thiol de formule $R_3SH$, dans laquelle $R_3$ est tel que défini ci-dessus, la réaction étant effectuée dans un solvant convenable, tel que le diméthylformamide, à une température comprise entre 0 et 50 °C et de préférence en présence d'un agent alcalin,

3) à éliminer, par des méthodes connues comme par exemple l'hydrolyse, les groupes protecteurs situés sur les fonctions amine et acide du produit obtenu,

4) éventuellement à transformer la fonction acide en sel ou ester par des procédés connus.

2. Procédé selon la revendication 1, caractérisé en ce que l'acide de formule IV est l'acide dans lequel $R_1$ et $R_2$ sont le groupe méthyle et en ce que le composé thiol mis en œuvre dans l'étape 2 est choisi parmi les composés de formule $R_3SH$ dans laquelle $R_3$ est l'un des groupes ci-après

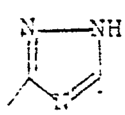 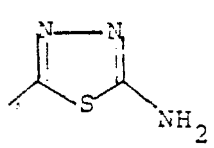 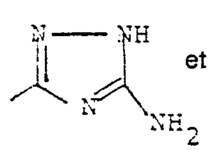 et 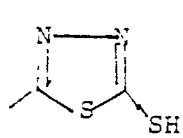

3. Procédé selon la revendication 1, caractérisé en ce que l'acide de formule IV est l'acide dans lequel $R_1$ et $R_2$ sont le groupe méthyle et en ce que le composé thiol mis en œuvre dans l'étape 2 est un composé de formule $R_3SH$ dans laquelle $R_3$ est le groupe

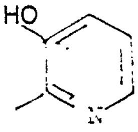

4. Procédé selon l'une quelconque des revendications 1 à 3, caractérisé en ce que les céphalosporines sont obtenus sous la forme d'isomère syn ou d'isomère anti ou sous forme d'un mélange de ces isomères.

**Claims** (for the Contracting States : BE, CH, DE, FR, GB, IT, LI, LU, NL, SE)

1. New cephalosporins, characterized in that they correspond to formula

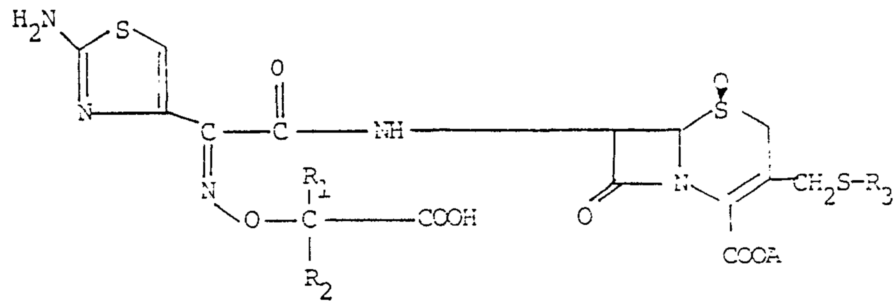

(II)

in which

$R_1$ and $R_2$, taken separately, each represent a methyl group or

$R_1$ and $R_2$ taken together represent a 1,3-propylene group,

$R_3$ represents a heterocycle of formula

a) 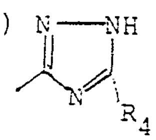 with $R_4$ = H, $NH_2$

b) 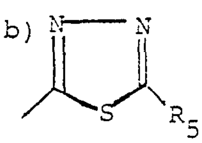 with $R_5$ = $NH_2$, SH

c) 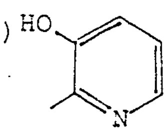

A represents hydrogen, a cation or an ester or hemiacetal, easily hydrolyzable or metabolically labile and pharmaceutically acceptable.

2. New cephalosporins according to claim 1, characterized in that they are in the form of syn isomer or anti isomer or in the form of a mixture of these isomers.

3. Drugs having antibacterial action, characterized in that they contain at least one new cephalosporin according to one of claims 1 and 2.

4. Drugs according to claim 3, characterized in that they contain a product of formula II in which $R_1$ and $R_2$ are $CH_3$, A is H and $R_3$ is selected from

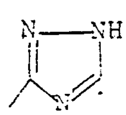 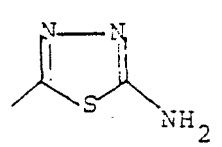 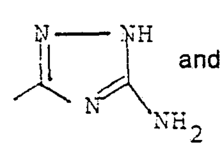 and 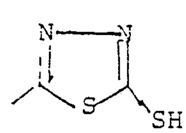

5. Drugs according to claim 3, characterized in that they contain a product of formula II in which $R_1$ and $R_2$ are $CH_3$, A is H and $R_3$ is

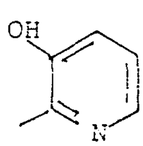

6. Process for preparing the new cephalosporins according to any one of claims 1 and 2, characterized in that it comprises the following successive reactions

— acylating the 7-amino 3-bromomethyl 3-cepheme carboxylate of tertiobutyl S-oxyde-1 on an acid of formula

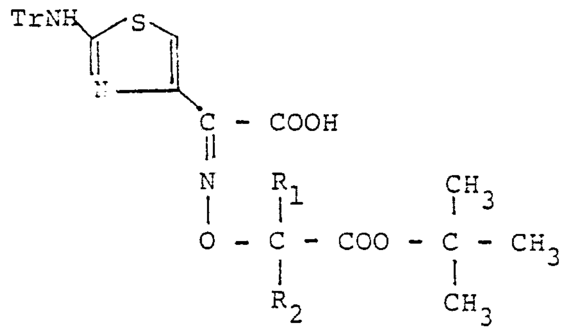

in which $R_1$ and $R_2$ have the meaning given in claim 1 and Tr is a protector group, for example trityl, said acid having been activated by conversion, for example into anhydride, said reaction of acylation being carried out in solution for example in dimethylformamide,

— then reacting the product obtained on a thiol of formula $R_3SH$, the reaction being carried out in a suitable solvent, such as dimethylformamide, at a temperature of between 0 and 50 °C and preferably in the presence of an alkaline agent,

— then removing, by known methods such as for example hydrolysis, the protector groups located on the amine and acid functions of the product obtained,

— then possibly converting the acid function into salt or ester by employibng known processes.

**Claims** (for the Contracting State AT)

1. Process for obtaining cephalosporins, corresponding to formula

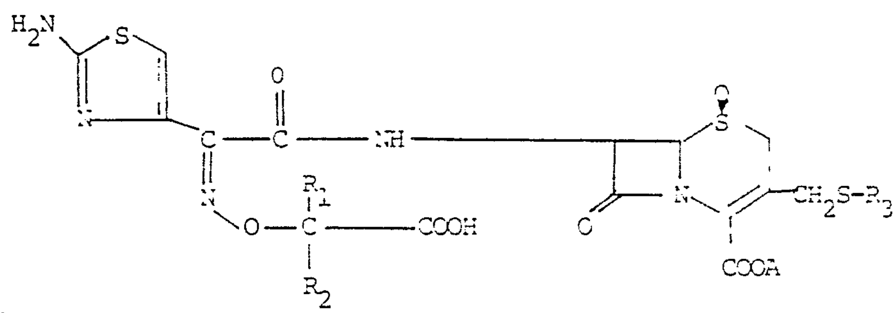

(II)

in which

$R_1$ and $R_2$, taken separately, each represent a methyl group or
$R_1$ and $R_2$ taken together represent a 1,3-propylene group,
$R_3$ represents a heterocycle of formula

a) 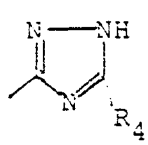 with $R_4$ = H, $NH_2$

b) 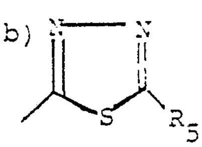 with $R_5$ = $NH_2$, SH

c) 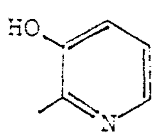

A represents hydrogen, a cation or an ester or hemiacetal, easily hydrolyzable or metabolically labile and pharmaceutically acceptable, characterized in that it consists in :

1) acylating the 7-amino 3-bromomethyl 3-cepheme carboxylate of tertiobutyl S-oxide-1 on an acid of formula

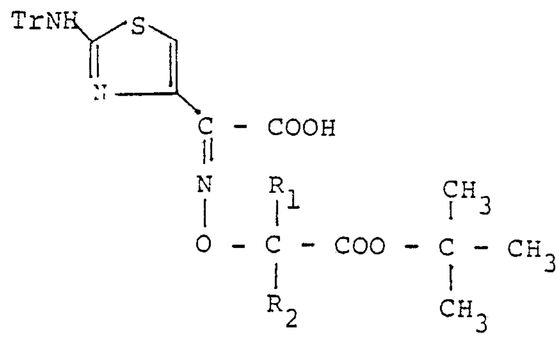

(IV)

in which $R_1$ and $R_2$ have the meaning given hereinabove and Tr is a protector group, for example trityl, said acid having been activated by conversion, for example into anhydride, said reaction of acylation being carried out in solution for example in dimethylformamide,

2) then reacting the product obtained on a thiol of formula $R_3SH$, in which $R_3$ is such as defined above, the reaction being carried out in a suitable solvent, such as dimethylformamide, at a temperature of between 0 and 50 °C and preferably in the presence of an alkaline agent,

3) then removing, by known methods such as for example hydrolysis, the protector groups located on the ȧmine and acid functions of the product obtained,

4) then possibly converting the acid function into salt or ester by employing known processes.

2. Process according to claim 1, characterized in that the acid of formula IV is the acid in which $R_1$ and $R_2$ are the methyl group and in that the thiol compound used in step 2 is selected from the compounds of formula $R_3SH$ in which $R_3$ is one of the following groups

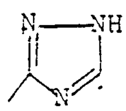 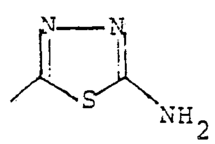 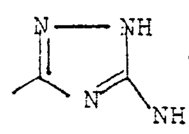 and 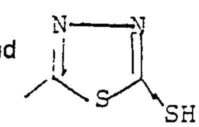

3. Process according to claim 1, characterized in that the acid of formula IV is the acid in which $R_1$ and $R_2$ are the methyl group and in that the thiol compound used in step 2 is a compound of formula $R_3SH$ in which $R_3$ is the group

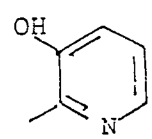

4. Process according to any one of claims 1 to 3, characterized in that the cephalosporins are obtained in the form of syn isomer or anti isomer or in the form of a mixture of these isomers.

**Patentansprüche** (für die Vertragsstaaten : BE, CH, DE, FR, GB, IT, LI, LU, NL, SE)

1. Neue Cephalosporine, dadurch gekennzeichnet, daß sie der Formel

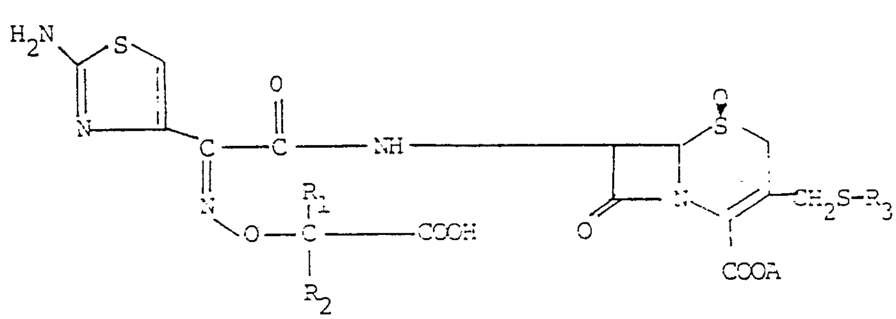

(II)

entsprechen, worin

$R_1$ und $R_2$, getrennt betrachtet, jeweils eine Methylgruppe darstellen, oder

$R_1$ und $R_2$, gemeinsam betrachtet, jeweils eine 1,3-Propylengruppe darstellen ;

$R_3$ einen Heterocyclus der Formel

a) 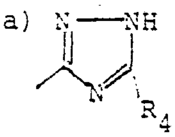 wobei $R_4$ = H, $NH_2$

b) 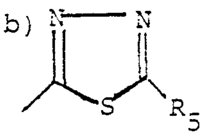 wobei $R_5$ = $NH_2$, SH

c) 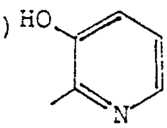

bedeutet

und A für Wasserstoff, ein Kation oder einen Ester oder ein leicht hydrolysierbares oder metabolisch labiles Hemiacetal, die pharmazeutisch akzeptabel sind, steht.

2. Neue Cephalosporine nach Anspruch 1, dadurch gekennzeichnet, daß sie in Form des syn-Isomeren oder anti-Isomeren oder in Form einer Mischung dieser Isomeren vorliegen.

3. Medikamente mit antibakterieller Wirkung, dadurch gekennzeichnet, daß sie mindestens ein neues Cephalosporin nach einem der Ansprüche 1 und 2 enthalten.

4. Medikamente nach Anspruch 3, dadurch gekennzeichnet, daß sie ein Produkt der Formel II enthalten, worin $R_1$ und $R_2$ $CH_3$ bedeuten, A H darstellt und $R_3$ ausgewählt is aus den folgenden Gruppen

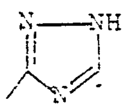 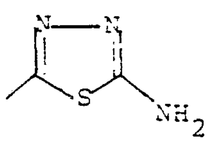 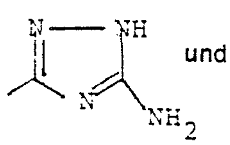 und 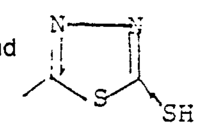

5. Medikamente nach Anspruch 3, dadurch gekennzeichnet, daß sie ein Produkt der Formel II enthalten, worin $R_1$ und $R_2CH_3$ bedeuten, A H darstellt und $R_3$

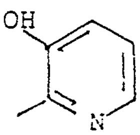

ist.

6. Verfahren zur Herstellung neuer Cephalosporine nach einem der Ansprüche 1 und 2, dadurch gekennzeichnet, daß man die folgenden Reaktionen nacheinander durchführt :

— man acyliert tert.Butyl-7-amino-3-brommethyl-cephem-4-carboxylat-1-S-oxid mit einer Säure der Formel

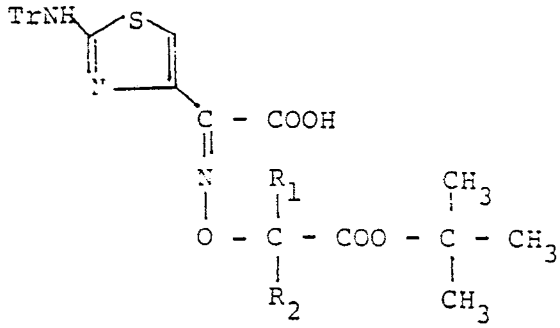

worin $R_1$ und $R_2$ die im Anspruch 1 angegebene Bedeutung haben und Tr eine Schutzgruppe, beispielsweise Trityl, ist, wobei die Säure durch Überführen, beispielsweise in das Anhydrid, aktiviert wurde und die Acylierungsreaktion in Lösung, beispielsweise in Dimethylformamid, durchgeführt wird,

— danach bringt man das erhaltene Produkt mit einem Thiol der Formel $R_3SH$ zur Umsetzung, wobei die Umsetzung in einem geeigneten Lösungsmittel, wie Dimethylformamid, bei einer Temperatur zwischen 0 und 50 °C und vorzugsweise in Gegenwart eines alkalischen Mittels durchgeführt wird,

— danach entfernt man mittels bekannter Verfahren, beispielsweise mittels Hydrolyse, die an den Amin- und Säurefunktionen des erhaltenen Produkts befindlichen Schutzgruppen,

— und danach führt man gegebenenfalls die Säurefunktion unter Anwendung bekannter Verfahren in das Salz oder den Ester über.

**Patentansprüche** (für den Vertragsstaat AT)

1. Verfahren zur Herstellung von Cephalosporinen der Formel

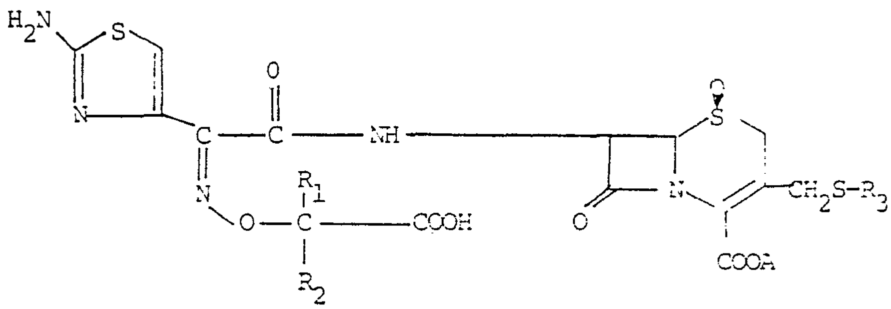

(II)

worin

$R_1$ und $R_2$, getrennt betrachtet, jeweils eine Methylgruppe darstellen, oder
$R_1$ und $R_2$, gemeinsam betrachtet, jeweils eine 1,3-Propylengruppe darstellen ;
$R_3$ einen Heterocyclus der Formel

a) 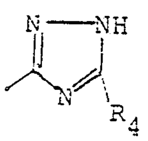 wobei $R_4$ = H, $NH_2$

b) 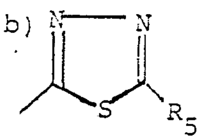 wobei $R_5$ = $NH_2$, SH

c) 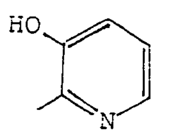

bedeutet

und A für Wasserstoff, ein Kation oder einen Ester oder ein leicht hydrolysierbares oder metabolisch labiles Hemiacetal, die pharmazeutisch akzeptabel sind, steht, dadurch gekennzeichnet, daß es darin besteht, daß

1) tert.Butyl-7-amino-3-brommethyl-cephem-4-carboxylat-1-S-oxid mit einer Säure der Formel

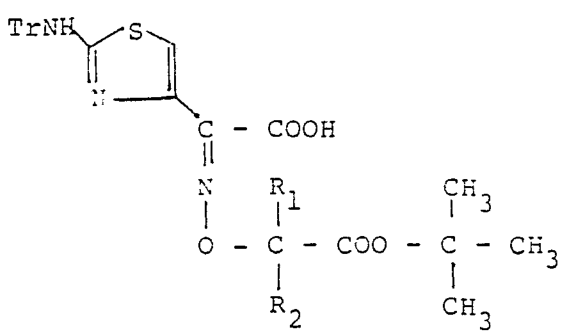

(IV)

worin $R_1$ und $R_2$ die oben angegebene Bedeutung haben und Tr eine Schutzgruppe, beispielsweise Trityl, ist, acyliert wird, wobei die Säure durch Überführen, beispielsweise in das Anhydrid, aktiviert wurde und die Acylierungsreaktion in Lösung, beispielsweise in Dimethylformamid, durchgeführt wird,

2) das erhaltene Produkt mit einem Thiol der Formel $R_3SH$, worin $R_3$ obige Bedeutung hat, zur Umsetzung gebracht wird, wobei die Umsetzung in einem geeigneten Lösungsmittel, wie Dimethylformamid, bei einer Temperatur zwischen O und 50 °C und vorzugsweise in Gegenwart eines alkalischen Mittels durchgeführt wird,

3) die an den Amin- und Säurefunktionen des erhaltenen Produkts befindlichen Schutzgruppen mittels bekannter Verfahren, beispielsweise mittels Hydrolyse, entfernt werden,

4) gegebenenfalls die Säurefunktion mittels bekannter Verfahren in das Salz oder den Ester übergeführt wird.

2. Verfahren nach Anspruch 1, dadurch gekennzeichnet, daß die Säure der Formel IV eine Säure ist, worin $R_1$ und $R_2$ eine Methylgruppe sind, und daß die in Stufe 2 eingesetzte Thiolverbindung ausgewählt ist aus den Verbindungen der Formel $R_3SH$, worin $R_3$ eine der nachstehenden Gruppen ist

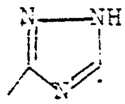 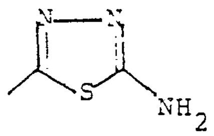 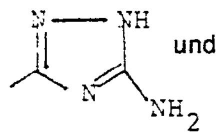 und 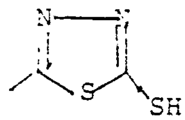

3. Verfahren nach Anspruch 1, dadurch gekennzeichnet, daß die Säure der Formel IV eine Säure ist, worin $R_1$ und $R_2$ eine Methylgruppe sind, und daß die in Stufe 2 eingesetzte Thiolverbindung eine Verbindung der Formel $R_3SH$ ist, worin $R_3$ die Gruppe

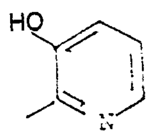

bedeutet.

4. Verfahren nach einem der Ansprüche 1 bis 3, dadurch gekennzeichnet, daß die Cephalosporine in syn-Isomeren-oder anti-Isomerenform oder in Form einer Mischung dieser Isomeren erhalten werden.